# EUROPEAN PATENT APPLICATION

(11) **EP 2 575 008 A1**
(43) Date of publication of application: **03.04.2013**
(21) Application number: 12186253.6
(22) Date of filing: 27.09.2012
(51) Int. Cl.: G06F 3/02, A61B 5/00, G06F 3/0354

(54) **Imaging apparatus for diagnosis**

(30) Priority: 28.09.2011 JP 2011211758
(71) Applicant: Terumo Kabushiki Kaisha, Tokyo (JP)
(72) Inventor: Horiike, Toyokazu, Fujinomiya, Shizuoka (JP)
(74) Representative: Dossmann, Gérard

(57) **Abstract**

An imaging apparatus (103) for diagnosis includes a display unit (113) for displaying a plurality of generated cross-sectional images sequentially in chronological order, first and second operation members (341,342) which are arranged on an operation panel (112) and which are for appending a bookmark to the cross-sectional image, and a control unit (111) for appending a bookmark to a cross-sectional image during display in response to a user operation toward at least one of the first and second operation members during the display of the cross-sectional image by the display unit, wherein the first operation member (341) is provided on the right side seen from the center portion in the right and left direction with respect to a user in a case in which the user directly-faces the operation panel, and the second operation member (342) is provided on the left side seen from the center portion of the operation panel. Therefore, regardless of whether the user's dominant hand is his/her right hand or left hand, it is possible to provide a good operability of the bookmark buttond (341, 342).

## Description

The present invention contains subject matter related to Japanese Patent Application JP 2011-211758 filed in the Japanese Patent Office on September 28, 2011, the entire content of which is incorporated herein by reference.

The present invention generally relates to an imaging apparatus for diagnosis.

In the past, for purposes of diagnosing arteriosclerosis, for performing diagnosis before a surgical operation at the time of intravascular diagnosis by a high-functional catheter such as a balloon catheter or a stent and the like, or to confirm the result after a surgical operation, there has been broadly utilized an imaging apparatus for diagnosis.

An example of a known representative imaging apparatus for diagnosis is an intravascular ultrasound (IVUS) diagnostic apparatus. Generally, the intravascular ultrasound (IVUS) diagnostic apparatus is an apparatus which radially operates an ultrasound probe unit, having a built-in transmitting and receiving unit composed of an ultrasound transducer (i.e., vibrator), inside a blood vessel; receives a reflected wave (ultrasonic wave echo), that is reflected by a biological tissue of a test subject, by the transmitting and receiving unit; and thereafter, visualizes a cross-sectional image of the blood vessel based on the intensity of the ultrasonic wave echo signal generated by applying a known process involving amplification, detection and the like.

Another example of a known imaging apparatus for diagnosis is an optical coherent tomography (OCT) apparatus which carries out image diagnosis by utilizing light coherency. The optical coherence tomography apparatus is an apparatus which emanates measurement light onto a blood vessel wall while rotating the transmitting and receiving unit in a state in which an optical probe unit having a built-in transmitting and receiving unit, that is mounted with an optical lens and an optical mirror at the distal end thereof, and also having a built-in optical fiber, is inserted into the inside of a blood vessel; carries out radial scan by light-receiving a reflected light from a biological tissue; and visualizes a cross-sectional image of the blood vessel based on interference light by making thus obtained reflected light and the reference light, which was divided from the measurement light beforehand, interfere with each other.

Further, in recent years, there has been developed also an optical frequency domain imaging (OFDI) apparatus utilizing wavelength sweep as an improved type of the optical coherence tomography apparatus. Regarding the optical frequency domain imaging (OFDI) apparatus utilizing wavelength sweep, the basic construction is similar as that of the optical coherent tomography (OCT) apparatus, but uses a light source having a longer wavelength compared with the optical coherent tomography (OCT) apparatus and also continuously emanates lights having different wavelengths. Then, by employing a construction in which reflected-light intensity of each point in the depth direction of the biological tissue is found out by frequency analysis of the interference light, mechanism for changing the optical path length of the reference light is made to be unnecessary.

In this specification hereinafter, the intravascular ultrasound (IVUS) diagnostic apparatus, the optical coherent tomography (OCT) apparatus and the optical frequency domain imaging (OFDI) apparatus utilizing wavelength sweep are generically and collectively referred to as an "imaging apparatus for diagnosis." Thus, such phrase as used in this specification encompasses each of the noted apparatus.

Generally, the plurality of cross-sectional images which are visualized by using such an imaging apparatus for diagnosis are images obtained while moving the probe unit inserted into the blood vessel along the blood vessel direction. Then, at the time of reproduction, by displaying these obtained cross-sectional images continuously at a predetermined frame rate in the order of the obtained time instants, it is possible to display the cross-sectional images like moving images.

In case of displaying the cross-sectional image like a moving image, a situation can arise in which a cross-sectional image appears that an individual would like to view or watch more carefully. The reproduction or display of the image should thus desirably be stopped once by a "pause" operation or the like. Thought has also been given to an idea in which, in order to make it easier to observe one or a plurality of images which are desired to be watched carefully afterward, bookmarks are appended to the corresponding cross-sectional images. The appending of the bookmark to the desired cross-sectional image is executed by pushing a bookmark button arranged on an operation panel at the time point when the desired cross-sectional image is reproduced during the reproduction period of the cross-sectional image.

In a case in which a plurality of cross-sectional images, in particular, such as moving images are reproduced continuously, the operation of the bookmark button must be executed immediately when the desired cross-sectional image is displayed so that promptness is required. A situation can occur in which the user observes the cross-sectional image by directly-facing the display screen and the operation panel, and operates the bookmark button if it is necessary. The position the bookmark button is provided with respect to the user who directly-faces the display screen effects on the operability of the user.

In particular, in recent years, there are increasing chances of displaying a graphical user interface (hereinafter, referred to as GUI) on a display apparatus and carrying out operations by a pointing device such as a trackball and a mouse. The trackball and the mouse are devices to be operated by a dominant hand and in order to make the operation of the pointing device and the prompt operation by the bookmark button, in which operations by the dominant hand are necessary, go together, it is necessary to consider the relation between the arrangement of the bookmark button and the user's dominant hand.

An imaging apparatus for diagnosis according to one aspect is provided with a construction that obtains a reflected signal from a biological tissue by moving a transmitting and receiving unit of a probe including the transmitting and receiving unit, which carries out signal transmission and reception continuously, in the axial direction while rotating inside a body lumen and which generates a plurality of cross-sectional images of the biological tissue in the axial direction based on the obtained reflected signal. The imaging apparatus for diagnosis includes a display unit for displaying the plurality of generated cross-sectional images sequentially in chronological order, first and second operation members which are arranged on an operation panel and which are for appending a bookmark to the cross-sectional image, and a control unit for appending a bookmark to a cross-sectional image during display in response to a user operation toward at least one of the first and second operation members during the display of the cross-sectional image by the display unit, wherein the first operation member is provided on the right side seen from the center portion in the right and left direction with respect to a user in a case in which the user directly-faces the operation panel, and the second operation member is provided on the left side as seen from the center portion of the operation panel.

The imaging apparatus for diagnosis is this configured in a way that improves the operability of the bookmark button.

According to another aspect, an imaging apparatus for diagnosis comprises a probe positionable in a living body and comprising a transmitting and receiving unit which continuously performs signal transmission and reception involving transmitting signals towards biological tissue in the living body and receiving reflected signals, a control unit operatively connected to the probe and configured to generate successive cross-sectional images of biological tissue in the living body based on the reflected signals received by the transmitting and receiving unit, a display configured to sequentially display in chronological order the successive cross-sectional images of the biological tissue, and an operation panel possessing left and right sides, forward and rearward sides, and a center portion positioned centrally relative to the left and right sides of the operation panel. The operation panel comprises a plurality of manually operable buttons arranged on the top surface of the operation panel and including a start button to start display of the successive cross-sectional images, a stop button to stop display of the successive cross-sectional images, and a pause button to pause display of the successive cross-sectional images at a particular one of the cross-sectional images. The operation panel also comprises a pair of manually operable image bookmark appending buttons operable to append a bookmark to at least one of the cross-sectional images, the pair of image bookmark appending buttons being arranged on the top surface of the operation panel, with the image bookmark appending buttons being spaced apart from one another so that one of the image bookmark appending buttons is positioned between the center portion of the operation panel and the right side of the operation panel, while the other of the image bookmark appending buttons is positioned between the center portion of the operation panel and the left side of the operation panel. The control unit is configured to append a bookmark to the at least one cross-sectional image displayed on the display when one of the pair of image bookmark appending buttons is manually operated during display of the at least one cross-sectional image on the display.

Another aspect involves a method of identifying a cross-sectional image of biological tissue The method involves viewing on a display a plurality of successive cross-sectional images of biological tissue produced on the basis of signals continuously transmitted toward the biological tissue and reflected off the biological tissue, with the display being operatively connected to an operation panel possessing a center portion positioned centrally relative to left and right sides of the operation panel, and the operation panel including first and second operation members spaced apart from one another and positioned so that the first operation member is located between the center portion and the left side of the operation panel and the second operation member is located between the center portion and the right side of the operation panel. The method additionally involves manually operating either one of the first and second operation members while the successive cross-sectional images are being displayed on the display, and appending a bookmark to the cross-sectional image displayed on the display whenever the first operation member is manually operated while the successive cross-sectional images are being displayed on the display and whenever the second operation member is manually operated while the successive cross-sectional images are being displayed on the display.

FIG. 1 is a perspective view showing an outward-appearance constitution of an imaging apparatus for diagnosis disclosed here.

FIGS. 2A and 2B are schematic cross-sectional views explaining a radial operation and a pull-back operation of a transmitting and receiving unit.

FIG. 3 is a view of one example of the configuration of an operation panel forming a part of the imaging apparatus for diagnosis.

FIG. 4 is a view showing one example of a display screen displayed on a monitor of the imaging apparatus for diagnosis.

FIG. 5 is a flowchart showing a cross-sectional image re-displaying process and a bookmark appending process.

Set forth below is a detailed description of one embodiment of the imaging apparatus for diagnosis representing an example of the imaging apparatus for diagnosis disclosed here.

### 1. Outward-Appearance Constitution of Imaging Apparatus for Diagnosis

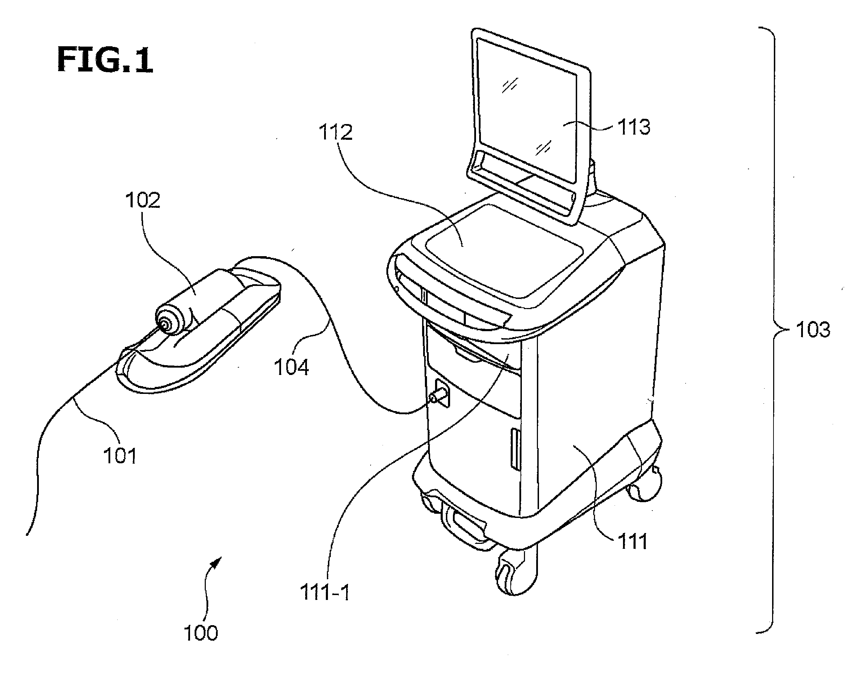

FIG. 1 is a view showing an outward-appearance constitution (construction or configuration) of an imaging apparatus for diagnosis (intravascular ultrasound diagnostic apparatus "hereinafter, referred to as IVUS apparatus", optical coherence tomography apparatus "hereinafter, referred to as OCT apparatus" or optical frequency domain imaging apparatus utilizing wavelength sweep (hereinafter, referred to as OFDI apparatus") 100 according to one embodiment.

As shown in FIG. 1, the imaging apparatus for diagnosis 100 includes a catheter unit 101, a scanner & pull-back unit 102 and an operation control apparatus 103. The scanner & pull-back unit 102 and the operation control apparatus 103 are connected by a signal line 104 (a wire in case of IVUS apparatus and an optical fiber in case of OCT or OFDI apparatus).

The catheter unit 101 is directly inserted inside a blood vessel (inside a body lumen) and measures a state of the blood vessel (biological tissue) by using a transmitting and receiving unit. The scanner & pull-back unit 102 is detachable with respect to the catheter unit 101 and is built-in with a linear drive motor and a radial & rotary drive motor. By driving the linear drive motor while driving this radial & rotary drive motor, the scanner & pull-back unit 102 carries out a pull-back operation (mentioned later) for linearly moving an ultrasound probe unit (IVUS apparatus) or an optical probe unit (OCT/OFDI apparatus), which includes a transmitting and receiving unit, inside the catheter unit 101 while carrying out the radial scan thereof.

For visualizing the cross-sectional image of the blood vessel, the operation control apparatus 103 operates or functions to, for example, output an ultrasound and a measurement light, input (receive or permit input of) various kinds of set values and the like, process data obtained by the measurement, and display the cross-sectional images resulting from such processing. In the operation control apparatus 103, there is provided a control unit 111 in which, for example, an ultrasound and a measurement light are outputted, data obtained by the measurement are processed, and the processed result is outputted. The imaging apparatus for diagnosis also includes a printer & DVD recorder 111-1 in which the processed result in the control unit 111 is printed, is stored as data and so on.

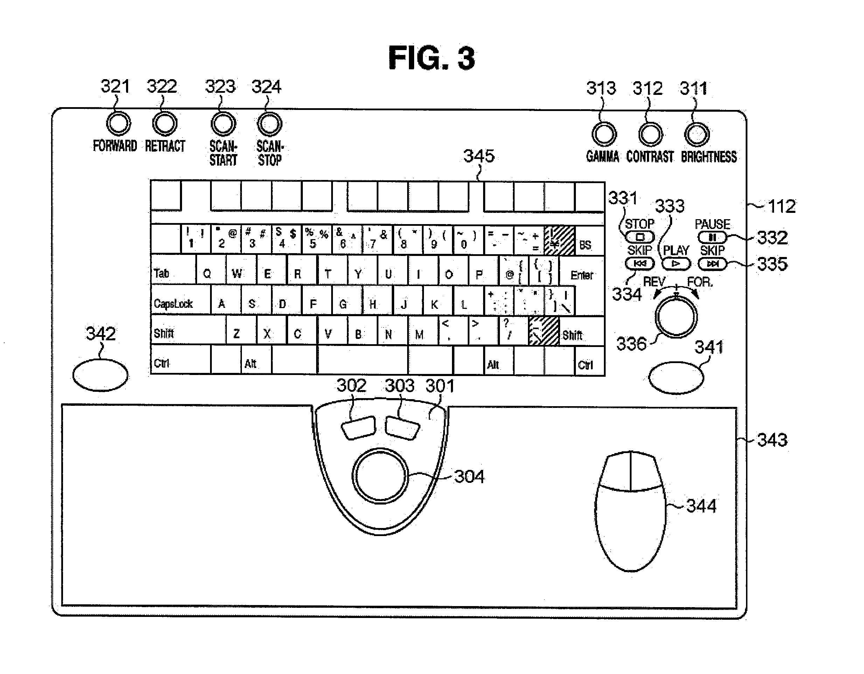

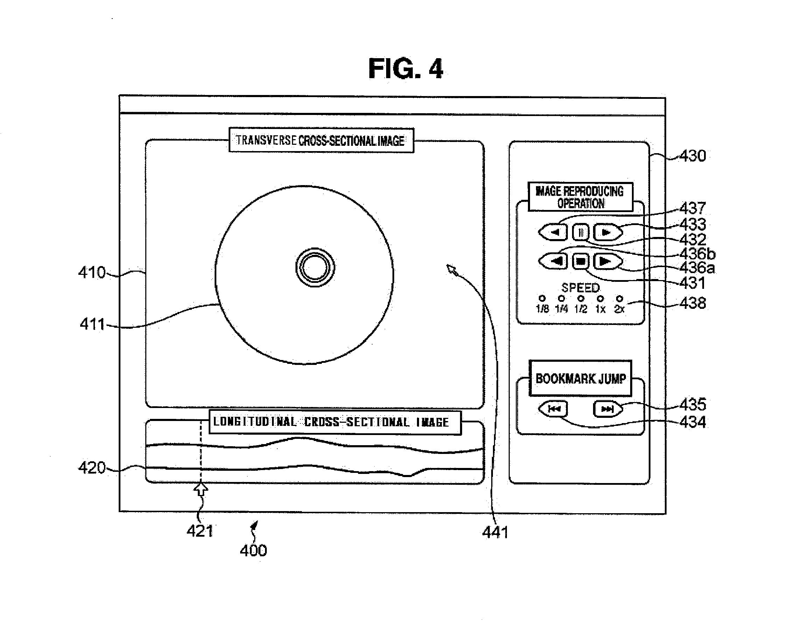

The imaging apparatus for diagnosis 100 includes an operation panel 112, and the user carries out inputs of various kinds of set values, inputs instructions for the measurement and the display, or the like through the operation panel 112. Details of the operation panel 112 will be described later with reference to FIG. 3. The imaging apparatus for diagnosis 100 also includes a LCD monitor 113 as a display apparatus that displays the processed result in the control unit 111. Details of the LCD monitor 113 will be described later with reference to FIG. 4.

### 2. Radial Operation of Ultrasound Probe Unit or Optical Probe Unit, and Pull-back Operation

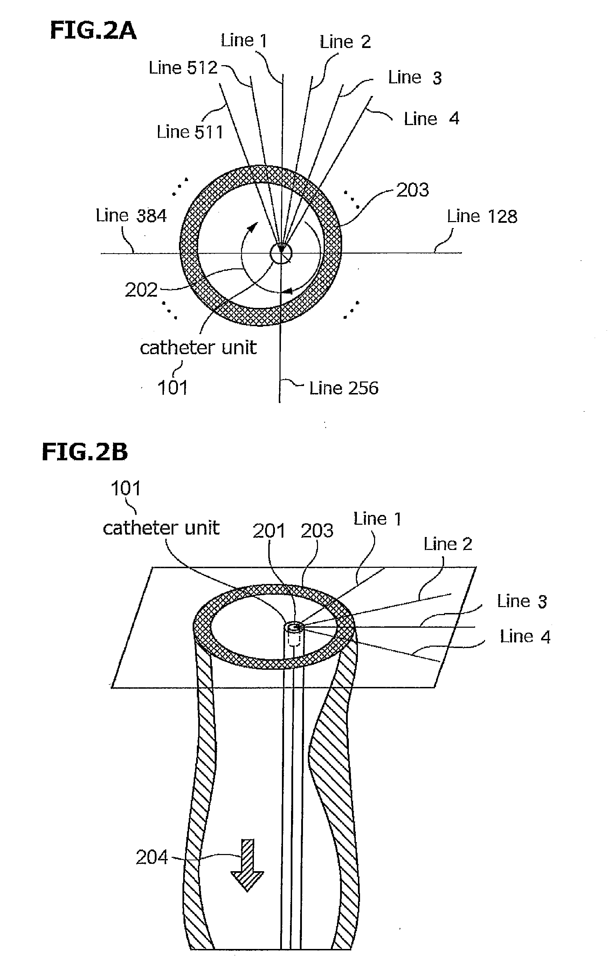

FIGS. 2A and 2B schematically illustrate a radial operation and a pull-back operation of an ultrasound probe unit or an optical probe unit inside the catheter unit 101 which is inserted inside a blood vessel. FIG. 2A illustrates a blood-vessel cross-section 203 into which the ultrasound probe unit or the optical probe unit is inserted. As mentioned above, the ultrasound probe unit or the optical probe unit is mounted with a transmitting and receiving unit 201 inside the distal end thereof and rotates toward an arrow 202 direction by a radial & rotary drive motor inside the scanner & pull-back unit 102.

In the transmitting and receiving unit 201, there is carried out transmission & reception of the ultrasound or the measurement light at each rotary angle. Lines 1, 2, ···, 512 indicate transmitting directions of the ultrasound or the measurement light at respective rotary angles. In the imaging apparatus for diagnosis 100 according to this embodiment, transmissions & receptions of the ultrasound or the measurement light are carried out 512 times intermittently during a time period in which the transmitting and receiving unit 201 rotates 360 degrees for a predetermined blood vessel cross-section 203. The number of times of the transmissions & receptions of the ultrasound or the measurement light during the rotation of 360 degrees is not limited by that number in particular, as the number can be set as desired.

Such a transmission & reception of the ultrasound or the measurement light is further carried out while the transmitting and receiving unit 201 advances inside the blood vessel (inside the catheter) along an arrow 204 direction shown in FIG. 2B. The movement of the transmitting and receiving unit 201 toward the arrow direction is carried out by a linear drive motor inside the scanner & pull-back unit 102. In this manner, by virtue of the pull-back operation for carrying out linear driving while also carrying out radial scan, a cross-sectional image is obtained at each position of the transmitting and receiving unit 201 and there are obtained a plurality of cross-sectional images along the blood-vessel direction.

### 3. Constitution of Operation Panel 112

FIG. 3 illustrates the configuration of the operation panel 112, including the arrangement of features on the operation panel 112. Generally speaking, the operation panel 112 possesses left and right sides (i.e., the sides toward the left and the right respectively as viewed in FIG. 3), and forward and rearward sides (i.e., the sides toward the bottom and the top respectively as viewed in FIG. 3). An LCD monitor operation unit 301 is a pointing device and is used for a purpose in which the user operates a cursor pointer displayed on the LCD monitor 113 and gives an instruction to the imaging apparatus for diagnosis 100 through a GUI (graphical user interface) offered by the LCD monitor 113. The operation panel also includes a trackball 304 used when moving the pointer displayed on the LCD monitor 113, and right and left click buttons 302, 303 respectively. Various kinds of settings (rotation speed and the like) at the time of diagnosis are carried out through the LCD monitor operation unit 301. Also, there is provided a mouse 344 other than the trackball 304 as a pointing device for carrying out an instruction input to the GUI by operating a cursor pointer. Therefore, there is provided a mouse pad 343 on the operation panel 112. The mouse pad 343 provides a mouse operation-area at the right and left areas on the near side of the operation panel 112 in order to make it easy for the user, who directly-faces the operation panel 112, to operate it by either his/her left hand and/or his/her right hand. In the example of FIG. 3, the mouse pad 343 provides areas continuous in the right and left direction, but it is possible for the areas on the left side and on the right side to be separated from each other.

The back right side of the operation panel 112 is with provided an operation switch group (311 to 313) for carrying out screen adjustments of the LCD monitor 113. The operation buttons include an operation button 311 for adjusting "brightness", an operation button 312 for adjusting "contrast", and an operation button 313 for adjusting "gamma".

The back left side of the operation panel 112 is provided with an operation switch group (321 to 324) for operating the scanner & pull-back unit 102 and the group is used when carrying out radial operation and pull-back operation of the transmitting and receiving unit 201 of the catheter unit 101. The switch group includes a forward button 321 which, during a time period in which this forward button 321 is pressed, the linear drive motor of the scanner & pull-back unit 102 operates and thus, the transmitting and receiving unit 201 inside the catheter unit 101 moves toward the distal direction inside the blood vessel (the operation stops when the pressing of the forward button 321 is released). The switch group also includes a retract button 322 which, during a time period in which the retract button 322 is pressed, the linear drive motor of the scanner & pull-back unit 102 operates and thus, the transmitting and receiving unit 201 inside the catheter unit 101 moves toward a direction opposite to the distal direction inside the blood vessel (the operation stops when the pressing of the retract button 322 is released).

The switch group further includes a scan-start button 323 by which, when the scan-start button 323 is pressed, the radial & rotary drive motor of the scanner & pull-back unit 102 rotates the transmitting and receiving unit 201 inside the catheter unit 101 at a predetermined rotation speed, and a scan-stop button 324 which, by pressing the scan-stop button 324, stops the rotation of the transmitting and receiving unit 201 stops.

On the right side of the operation panel 112 there is provided an operation switch group (331 to 336) which is used when a cross-sectional image stored in a memory is displayed on the LCD monitor 113. The operation switch group includes a "PLAY" button 333 to display the cross-sectional image stored in the memory on the LCD monitor 113 at a predetermined frame rate. A "STOP" button 331 is used to stop the display of the cross-sectional image, which was started by the "PLAY" button 333. A "PAUSE" button 332 is used for instantly-stopping the display of the cross-sectional image at the predetermined frame rate. That is, operation of the "PLAY" button causes the cross-sectional images stored in the memory to be displayed, one at a time, on the LCD monitor 113 at a predetermined frame rate. While cross-sectional images are being displayed on the LCD monitor following operation of the "PLAY" button, the operation of the "STOP" button 331 stops the display of cross-sectional images so that cross-sectional images are not displayed on the LCD monitor. And while cross-sectional images are being displayed on the LCD monitor following operation of the "PLAY" button, the operation of the "PAUSE" button 332 instantly-stops the display of the cross-sectional image so that the displayed cross-sectional image remains displayed on the LCD display.

A "SKIP" button 334 is used for skipping the cross-sectional image during display for a bookmarked cross-sectional image lying at a previous position seen from the position now displayed. A "SKIP" button 335 is used for skipping the image for a bookmarked cross-sectional image which is lying at a rear position seen from the position now displayed. A fast-forward & rewind button 336 is provided, and by turning the button 336 clockwise, successive cross-sectional images are displayed in a fast-forward mode. Also, by turning the button 336 anticlockwise or counterclockwise, successive cross-sectional images are displayed in a rewind mode.

The operation panel 112 further includes a first operation member 341 and a second operation member 342, both of which constitute image bookmark appending members or buttons arranged on the upper surface of the operation panel and operable to append a bookmark (bookmarker) to one or more of the cross-sectional images. Hereinafter, these buttons are described as a first bookmark button 341 and a second bookmark button 342. When a desired cross-sectional image is displayed on the LCD monitor 113, it is possible to append or apply the bookmark to the displayed cross-sectional image by pressing down either one of these bookmark buttons 341, 342. It is possible for the cross-sectional image with the bookmark appended thereto to be searched and displayed by the abovementioned "SKIP" buttons 334, 335. The operation panel 112 also includes a keyboard 345 used to input various kinds of characters. The first bookmark button 341 and the second bookmark button 342 are provided on the right side and on the left side respectively of the center portion in the right and left direction with respect to a user who is directly facing the operation panel 112.

### 4. Display Screen Displayed on LCD Monitor of Imaging Apparatus for Diagnosis

The description which follows explains a display screen displayed on the LCD monitor 113 of the imaging apparatus for diagnosis 100. FIG. 4 shows one example of a display screen 400 for displaying a transverse cross-sectional image and a longitudinal cross-sectional image in an axial direction on the LCD monitor 113 of the imaging apparatus for diagnosis 100.

As shown in FIG. 4, the display screen 400 is provided with a transverse cross-sectional image display area 410 in which the transverse cross-sectional image in the direction perpendicular to the axis of the blood vessel is displayable continuously, a longitudinal cross-sectional image display area 420 in which the longitudinal cross-sectional image in the axial direction which is reconstructed from line data of that transverse cross-sectional image, and an operation area 430 in which is provided a GUI for carrying out various kinds of operations in the display screen 400.

In the operation area 430, there is arranged an operation switch group which is used when the cross-sectional image is displayed on the LCD monitor 113 and according to the selection thereof by operating a mouse cursor 441 using the abovementioned pointing device, a similar function as that of the operation buttons 331 to 336 on the abovementioned operation panel 112 can be realized. More specifically, the operation buttons 431, 432, 433, 434, 435 in the operation area 430 have a function equivalent to that of the "STOP" button 331, the "PAUSE" button 332, the "PLAY" button 333 and the "SKIP" buttons 334, 335 respectively on the abovementioned operation panel 112. Also, an operation button 436a has a "fast-forward" function like that of the fast-forward & rewind button 336, and an operation button 436b has a "rewind" function like that of the fast-forward & rewind button 336.

The user interface provided by the LCD monitor 113 in this embodiment disclosed as an example includes a further function which relates to the reproducing operation of the cross-sectional image and which does not exist on the operation panel 112. That is, a reverse-direction "PLAY" button 437 is used to carry out the reproduction in a reverse direction (so as to go back in terms of time) opposite to the "PLAY" button 433. It is possible for a speed setting 438 to set the reproduction speed to be lower speeds (118 to 1/2) with respect to the normal speed (1x) or to be a higher speed (2x) with respect to the normal speed (1x).

Through use or operation of the abovementioned operation area 430, it is thus possible to, for example, continuously display the transverse cross-sectional images in the transverse cross-sectional image display area 410, to instantly-stop at any one of the transverse cross-sectional images currently displayed, and to also continuously display the plurality of transverse cross-sectional images in a fast-forward or rewind mode. Also, in the longitudinal cross-sectional image display area 420, the position in the axial direction of the transverse cross-sectional image which is displayed at present in the transverse cross-sectional image display area 410 is indicated by an arrow 421. Therefore, the position of the arrow 421 moves in accordance with the progress of the display of the transverse cross-sectional image 411 in the transverse cross-sectional image display area 410.

### 5. Explanation of Bookmark Appending Process

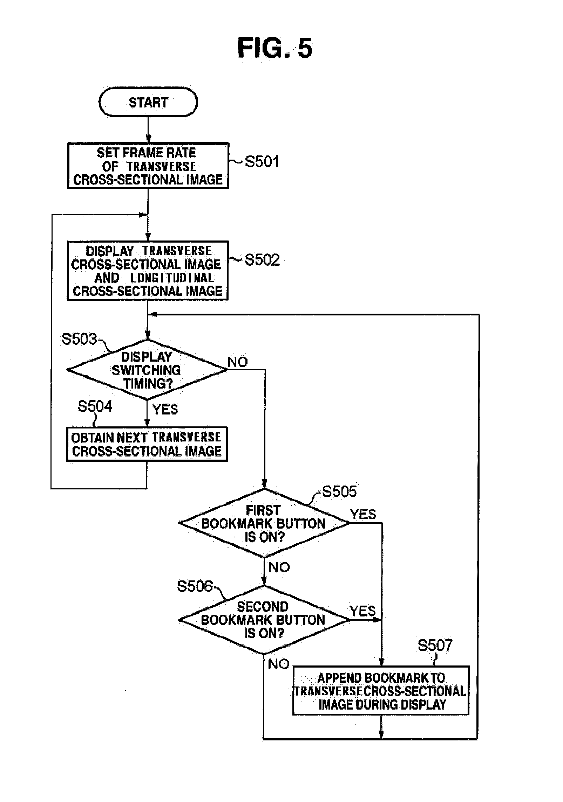

Set forth next is a description of the bookmark appending process or operation using the first bookmark button 341 and/or the second bookmark button 342. FIG. 5 is a flowchart explaining a bookmark appending process according to this exemplified embodiment.

In step S501, the control unit 111, or a part thereof, of the imaging apparatus for diagnosis 100 sets a frame rate at which the transverse cross-sectional images are displayed on the display area 410 of the display screen 400. The control unit can set the frame rate based on the speed setting set by the user at the speed setting 438. If "1x" is selected in the speed setting 438, a default frame rate is selected. Also, for example, if "2x" is selected in the speed setting 438, a frame rate having twice the default frame rate is set. Similarly, if "1/4" is selected, a frame rate having one quarter of the default frame rate is set.

In step S502, the control unit displays the transverse cross-sectional image, which is read-out from the memory, on the transverse cross-sectional image display area 410 of the LCD monitor 113, displays the longitudinal cross-sectional image, which is read-out from the memory, on the longitudinal cross-sectional image display area 420, and concurrently displays the arrow 421 on the longitudinal cross-sectional image display area 420 identifying the longitudinal location of the displayed transverse cross-sectional image. In step S503, it is judged whether or not it is the changeover timing of the image corresponding to the frame rate which was set in step S501 and if it is judged that it is the changeover timing, the process proceeds to step S504. That is, it is determined at step S503 whether the next successive transverse cross-sectional image should be displayed and if so, the routine moves to step S504. In step S504, the control unit obtains the next transverse cross-sectional image from the memory and then in step S502 displays this image on the image display area 410. In this manner, the transverse cross-sectional images are displayed on the LCD monitor 113 sequentially in time-series.

On the other hand, when it is judged in step S503 that it is not time to changeover to the next image, the process proceeds to step S505. In step S505, it is judged whether or not the first bookmark button 341 is ON.
In a case in which the first bookmark button is OFF, it is judged in step S506 whether or not the second bookmark button 342 is ON. In a case in which both of the first bookmark button 341 and the second bookmark button 342 are OFF, the process returns to step S503. On the other hand, in a case in which at least either one of the first bookmark button 341 and the second bookmark button 342 is ON, the process proceeds to step S507. In step S507, the control unit appends a bookmark to the attribute information of the transverse cross-sectional image during display. At the same time, a mark showing the bookmark position is displayed at a corresponding position of the longitudinal cross-sectional image. Thereafter, the process returns to step S503.

When users press down and release the bookmark button, such action may be performed relatively slowly which may be recognized as multiple pushing operations. It is possible to employ procedures, involving for example monitoring the rising time of the button, to prevent bookmarks from being applied all together with respect to a plurality of transverse cross-sectional images by a single press-down operation of the bookmark button.

In the imaging apparatus for diagnosis 100 of this embodiment disclosed as an example, the first bookmark button 341 is arranged in the area on the right hand side of the user when the user directly-faces the operation panel 112 and the second bookmark button 342 is arranged in the area on the left hand side of the user when the user directly-faces the operation panel 112. Therefore, regardless of whether the user's dominant hand is his/her right hand or left hand, it is possible to provide a good operability.

Also, the trackball 304 as a pointing device is arranged between the first bookmark button 341 and the second bookmark button 342 which are arranged as mentioned above. Therefore, if the user is a right-hander, it is possible for him/her to operate the second bookmark button 342 by his/her left hand while operating the trackball 304 by his/her right hand. Similarly, if the user is a left-hander, it is possible for him/her to operate the first bookmark button 341 by his/her right hand while operating the trackball 304 by his/her left hand. In this manner, regardless of whether the user is a left-hander or a right-hander, it is possible to carry out the operation without crossing the hand for operating the trackball and the hand for operating the bookmark button, so that the operability thereof improves.

Also, the mouse pad 343 which is the area for operating the mouse 344 is provided on the near side (on the side of the user directly-facing the operation panel 112) of the operation panel 112 as an area which is continuous over the right and left directions of the operation panel 112. That is, the mouse pad 343 extends from the left side of the operation panel 112 to the right side of the operation panel 112 in an uninterrupted manner. Providing this relatively wide area for operating the mouse improves the operability of the mouse 344 and makes it usable for both a right-hander and a left-hander. Also, in case of carrying out the operation for appending a bookmark while operating the mouse 344 by a right hand, it is possible to prevent the user's hands from crossing each other by operating the mouse 344 in the area on the right side of the mouse pad 343 and by operating the second bookmark button 342 by his left hand. Similarly, in case of carrying out the operation for appending a bookmark while operating the mouse 344 by a left hand, it is possible to prevent the user's hands from crossing each other by operating the mouse 344 in the area on the left side of the mouse pad 343 and by operating the first bookmark button 341 by his right hand.

The mouse pad 343 is configured to be a pad which is continuous in the right and left directions, but the operation panel is not limited to this configuration. For example, it is possible that the area for operating the mouse 344 by a right hand and the area for operating the mouse 344 by a left hand are arranged so as to be separated from each other.

It is also possible to omit the LCD monitor operation unit 301 which includes the trackball 304 so that only the mouse 344 is used. Alternatively or conversely, it is possible to employ a construction in the operation panel 112 in FIG. 3 in which the mouse pad 343 is omitted and only the trackball 304 is provided.

The operation panel 112 is illustrated as having a construction in which two bookmark buttons are arranged on the operation panel 112. But the operation panel 112 is not limited in this regard. A plurality of bookmark buttons are preferably provided and arranged so that if the operator/user tries to operate a bookmark button and a pointing device simultaneously, the construction or configuration of the operation panel 112 allows them to be comfortably without causing the arms of the operator/user to cross each other or the like. FIG. 3 illustrates an example of one possible arrangement of the features, but other arrangements are possible.

The detailed description above describes features and aspects of embodiments of a imaging apparatus for diagnosis and manner of use/operation of an imaging apparatus for diagnosis. The invention is not limited, however, to the precise embodiments and variations described. Various changes, modifications and equivalents could be effected by one skilled in the art without departing from the spirit and scope of the invention as defined in the appended claims. It is expressly intended that all such changes, modifications and equivalents which fall within the scope of the claims are embraced by the claims.

## Claims

1. An imaging apparatus for diagnosis comprising:
a probe comprising a transmitting and receiving unit which carries out signal transmission and reception continuously;
a control unit operatively connected to the probe and configured to generate a series of cross-sectional images of biological tissue based on reflected signals obtained by the transmitting and receiving unit;
a display unit configured to display, sequentially in chronological order, the series of cross-sectional images;
an operation panel comprising first and second operation members operable to append bookmarks to at least one of the cross-sectional images in the series;
the control unit being configured to append a bookmark to the at least one cross-sectional image in response to a user operation of at least one of the first and second operation members during display of the at least one cross-sectional image on the display unit; and
the first operation member is provided on a right side of a center portion on the operation panel with respect to a user directly-facing the operation panel and the second operation member is provided on a left side of the center portion on the operation panel.

2. The imaging apparatus for diagnosis according to Claim 1,
wherein the display unit is a user interface operable by a mouse, and an area for operating the mouse exists on both the right side and the left side of a center portion of the operation panel.

3. The imaging apparatus for diagnosis according to Claim 2,
wherein the area for operating the mouse is provided on a front portion of the operation panel and extends continuously in an uninterrupted manner over the right side and the left side of the operation panel.

4. The imaging apparatus for diagnosis according to Claim 1,
wherein the display unit is a user interface operable by a trackball, and there is provided, at the operation panel, with the trackball as a pointing device and the trackball is provided between the first operation member and the second operation member with relation to right and left directions of the operation panel.

5. The imaging apparatus for diagnosis according to Claim 4,
wherein the trackball is located in the center portion of the operation panel.

6. The imaging apparatus for diagnosis according to Claim 1,
wherein the operation panel also comprises a keyboard that includes alphabetic ad numeric keys.

7. A method of operating an imaging apparatus for identifying a cross-sectional image of biological tissue comprising:
viewing on a display a plurality of successive cross-sectional images of biological tissue produced on the basis of signals continuously transmitted toward the biological tissue and reflected off the biological tissue, the display being operatively connected to an operation panel possessing a center portion positioned centrally relative to left and right sides of the operation panel, the operation panel including first and second operation members spaced apart from one another and positioned so that the first operation member is located between the center portion and the left side of the operation panel and the second operation member is located between the center portion and the right side of the operation panel;
manually operating either one of the first and second operation members while the successive cross-sectional images are being displayed on the display; and
appending a bookmark to the cross-sectional image displayed on the display whenever the first operation member manually is operated while the successive cross-sectional images are being displayed on the display and whenever the second operation member is manually operated while the successive cross-sectional images are being displayed on the display.

8. The method according to Claim 7, wherein the manual operation of either one of the first and second operation members comprises:
operating the first operation member while the successive cross-sectional images are being displayed on the display to append a bookmark to a first cross-sectional image displayed on the display when the first operation member is manually operated; and
operating the second operation member while the successive cross-sectional images are being displayed on the display to append a bookmark to a second cross-sectional image displayed on the display when the second operation member is manually operated.

9. The method according to Claim 7, wherein the manual operation of either one of the first and second operation members comprises:
manually operating the first operation member with a right hand while the successive cross-sectional images are being displayed on the display to append a bookmark to a first cross-sectional image displayed on the display when the first operation member is manually operated; and
manually operating the second operation member with a right hand while the successive cross-sectional images are being displayed on the display to append a bookmark to a second cross-sectional image displayed on the display when the second operation member is manually operated.

10. The method according to Claim 7, wherein the display is a graphical user interface, and the operation panel includes an area for operating a mouse, the mouse being operatively associated with the graphical user interface, the method further comprising:
operating the mouse with a user's left hand; and
the manual operation of either one of the first and second operation members comprising manually operating the first operation member with the user's right hand while the successive cross-sectional images are being displayed on the display to append a bookmark to a first cross-sectional image displayed on the display when the first operation member is manually operated, the first operation member being manually operated with the user's right hand at the same time as the mouse is operated with the user's left hand.

11. The method according to Claim 7, wherein the display is a graphical user interface, and the operation panel includes an area for operating a mouse, the mouse being operatively associated with the graphical user interface, the method further comprising:
operating the mouse with a user's right hand; and
the manual operation of either one of the first and second operation members comprising manually operating the first operation member with the user's left hand while the successive cross-sectional images are being displayed on the display to append a bookmark to a first cross-sectional image displayed on the display when the first operation member is manually operated, the first operation member being manually operated with the user's left hand at the same time as the mouse is operated with the user's right hand.

12. The method according to Claim 7, wherein the display is a graphical user interface, and the operation panel includes a trackball, the trackball being operatively associated with the graphical user interface, the method further comprising:
operating the trackball with a user's right hand; and
the manual operation of either one of the first and second operation members comprising manually operating the first operation member with the user's left hand while the successive cross-sectional images are being displayed on the display to append a bookmark to a first cross-sectional image displayed on the display when the first operation member is manually operated, the first operation member being manually operated with the user's left hand at the same time as the trackball is operated with the user's right hand.

13. The method according to Claim 7, wherein the display is a graphical user interface, and the operation panel includes an area for operating a trackball, the trackball being operatively associated with the graphical user interface, the method further comprising:
operating the trackball with a user's left hand; and
the manual operation of either one of the first and second operation members comprising manually operating the first operation member with the user's right hand while the successive cross-sectional images are being displayed on the display to append a bookmark to a first cross-sectional image displayed on the display when the first operation member is manually operated, the first operation member being manually operated with the user's right hand at the same time as the trackball is operated with the user's left hand.
